# EUROPEAN PATENT SPECIFICATION

(11) **EP 1 835 871 B1**
(45) Date of publication and mention of the grant of the patent: **22.05.2013**
(21) Application number: 05855170.6
(22) Date of filing: 22.12.2005
(51) Int. Cl.: A61B 5/055, A61M 13/00, G01R 33/28

(54) **SYSTEM, IMAGING SUITE, AND METHOD FOR USING AN ELECTRO-PNEUMATIC INSUFFLATOR FOR MAGNETIC RESONANCE IMAGING**
SYSTEM, BILDGEBUNGSSUITE UND VERFAHREN ZUR VERWENDUNG EINES ELEKTROPNEUMATISCHEN INSUFFLATORS FÜR DIE MAGNETRESONANZTOMOGRAPHIE
SYSTEME, SUITE D'IMAGERIE, ET PROCEDE D'UTILISATION D'UN INSUFFLATEUR ELECTRO-PNEUMATIQUE POUR IMAGERIE PAR RESONANCE MAGNETIQUE

(30) Priority: 22.12.2004 US 638643 P
(43) Date of publication of application: 26.09.2007
(73) Proprietor: Bracco Diagnostics Inc., Princeton NJ 08540 (US)
(72) Inventor: WILLIAMS, Robert, C., Jr., Fort Salonga, NY 11768 (US)
(74) Representative: Ravizza, Claudio
(86) International application number: PCT/US2005/046561
(87) International publication number: WO 2006/069231

(56) References cited:
- US-A- 3 177 871
- US-A- 5 487 376
- US-A- 6 136 292
- US-A1- 2001 037 063
- US-A1- 2001 044 576
- US-A1- 2002 169 415
- US-A1- 2002 169 415
- US-A1- 2006 079 758
- US-B1- 6 400 157

## Description

### FIELD OF THE INVENTION

The invention relates to a suite and system associated with using an electro-pneumatic insufflator with Magnetic Resonance Imaging (MRI).

### BACKGROUND OF THE INVENTION

Colorectal cancer, cancer of the large intestine and rectum, is second only to lung cancer in the amount of cancer deaths caused each year. Approximately 5% of all people will develop colorectal cancer within their lifetime. As is true with many other cancers, early detection of colon cancer or its precursors greatly increases chances of survival.

Precancerous polyps begin to form in the colon when cells in the lining of the intestine mutate and begin dividing rapidly. If left untreated, 8 to 12 percent of polyps will become cancerous tumors. Polyps sometimes bleed, and there may be some noticeable rectal bleeding that leads to early detection of precancerous growths. However, most of the time, this blood is invisible to the naked eye and is only detectable microscopically.

Gastrointestinal imaging can be used to accurately identify precancerous polyps and can thereby be used to prevent the development of colorectal cancer. The diagnostic performance of gastrointestinal imaging, including but not limited to CT imaging and magnetic resonance imaging (MRI), may be facilitated by distending a desired body part prior to and during the diagnostic procedure. Ideally, distention is maintained throughout the procedure to obtain the most accurate image. Currently, it is known to distend the colon or other body parts of an individual prior to and during examination by direct connection of an insufflator to the proximal end of a rectal catheter inserted into the rectum of the individual. With this device, air or carbon dioxide (CO₂), for example, can be introduced into the colon.

Currently, the practice of using an electro-mechanical insufflator to comfortably control distension of the colon with carbon dioxide for radiographic imaging of the colon, typically referred to as virtual colonoscopy, is limited to computed tomography (CT). Distending the colon with a gaseous media during such diagnostic procedures to open the colon's lumen provides a high to low contrast boundary defining its interior surface when exposed to X-rays. The radiologist can then view the resulting surface image in either 2-D or 3-D post scan to identify anatomic abnormalities, such as pre-cancerous growths, on the surface of the colon that could potentially represent a disease state in the colon. Since CT procedures rely on ionizing radiation or X-rays to produce images of the colon, the current design and construction of CT X-ray equipment is electrically and magnetically compatible with a wide variety of electro-mechanical equipment such as an insufflator or a contrast injector.

One product that has been used to comfortably control distension of the colon for CT procedures for virtual colonoscopy is the PROTOCO₂L™ system, available from E-Z-EM. The PROTOCO₂L™ system comprises an insufflator, consumable administration set, and an accessory cart. The insufflator unit sits atop the accessory cart where it can be wheeled directly into the CT room directly adjacent to the CT gantry (X-ray unit) and patient. The accessory cart also provides support to the replaceable carbon dioxide supply cylinders. The connection from the insufflator to the patient's rectum is made with the single use consumable administration set. The operator of the insufflator and CT equipment performs the procedure of making the patient connection and operates the insufflator controls next to the patient in the CT room.

As the speed and sophistication of magnetic resonance imaging (MRI) technology evolves, this sectional imaging modality is increasingly being used for virtual imaging of the colon since it is safer than CT imaging and can produce comparable image quality to that of a CT scan. In an MRI procedure, the patient is subject to relatively safe magnetic and radio frequency (RF) energy, whereas a CT scan utilizes ionizing radiation. As in a CT scan, the colon needs to be distended with a gaseous media during the MRI. In addition, the same benefits regarding the comfort and control provided by electro-pneumatic carbon dioxide distention of the colon for CT imaging can be maintained in magnetic resonance imaging.

The biggest component in an MRI system or machine is the magnet. The magnet in an MRI scanner has an extremely strong magnetic field, typically at least 10,000 times stronger than Earth's magnetic field (e.g., 0.5-2.0 Tesla vs. 0.5 micro-Tesla). Because of the power of these magnets, a room containing an MRI scanner can be very dangerous if precautions are not taken. Unsecured metal objects such as keys, scissors, or other ferrous objects can be pulled out of pockets and toward the opening of the magnet, which is also where the patient is placed during the imaging process. Mop buckets, IV poles, oxygen tanks, and many other objects have all been pulled into the magnetic fields of MRI scanners. It is also important to note that the magnetic force exerted on an object increases exponentially the closer it gets to the magnet, and the same is true with respect to the increasing size of an object - smaller objects could be pulled off the magnet by hand, but larger objects would may require additional force to remove.

Because of the power of MRI magnets, severe safety and functional implications would result if a clinician used an insufflator in an MRI imaging suite. Current insufflators include ferromagnetic materials for various components, most notably the iron cores associated with the electro-magnetic valves and the carbon dioxide supply cylinders themselves. Introducing these components in direct proximity to the strong magnetic field of the magnetic resonance (MR) scanner could result in a catastrophic interaction.

While it may be theoretically possible to substitute non-ferromagnetic materials for a number of the ferromagnetic materials of the current system, it would require a complete redesign of the pneumatic technology, which would be expensive and time-consuming. The electro-pneumatic insufflator relies on electro-magnetic on/off and proportional valves to accurately measure carbon dioxide volume and control the pressure and associated flow rate of the gas supplied to the patient. Such electro-magnetic valve technology has been in use for decades and has proven itself to be efficient, economical, and highly reliable. Even if it were possible to replace all ferromagnetic materials in the electro-magnetic valves, the introduction of any transient electrical signal in a shielded MR scanner room to control the resultant pneumatic system could potentially interfere with the RF signals generated within the MR scanner. Electrical transients created by the control electronics of a device such as an insufflator inside of a shielded MR scanner room, even if it were magnetically shielded itself, would be considered as a noise producer having the potential to interfere with the imaging acquisition. Such noise, even if minimal, could distort the resulting images to be interpreted by the clinician.

Document US6136292 discloses a device for insufflation of the colon with an insufflation medium via the colon for MRI imaging. This document implicitly discloses an insufflator and at least one room in which the imaging is performed. The document discloses a device according to the preamble of claim 16.

The invention is defined by claim 1 and 16. Further embodiments are described by the dependent claims.

Embodiments of the present invention may help provide an improved electro-pneumatic insufflator for magnetic resonance imaging based virtual colonoscopy. As such, the present invention may solve many of the problems identified by prior techniques and provide additional advantages.

### BRIEF DESCRIPTION OF THE DRAWINGS

Reference will now be made to the accompanying drawings, which are not necessarily drawn to scale, and wherein:
**FIG. 1** is a non-limiting illustration of an embodiment of the present invention that shows a MRI suite;
**FIG. 2** is a non-limiting illustration of one alternative embodiment of the present invention that shows a system for distending an organ is that is in accordance with at least one aspect of the invention;
**FIG. 3** is a perspective view of a non-limiting illustration of one alternative embodiment of the present invention that shows a system for distending an organ that is in accordance with at least one aspect of the invention;
**FIGS. 4A****,** **4B**, and **4C** are perspective drawings of a non-limiting illustration of one alternative embodiment of the present invention that shows a support stand that is in accordance with at least one aspect of the invention;
**FIG. 5** is a non-limiting illustration of one alternative embodiment of the present invention that shows a MRI suite and distension system as they are being used;
**FIG. 6** is a graph and associated dialogue in report form demonstrating the feasibility of one alternative embodiment of the present invention as explained in the Examples below.

### DETAILED DESCRIPTION OF THE INVENTION

The present invention now will be described more fully hereinafter with reference to the accompanying drawings, in which preferred embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art. Like numbers refer to like elements throughout.

The invention relates to an electro-pneumatic insufflator for magnetic resonance imaging based virtual gastrointestinal imaging. Virtual gastrointestinal imaging may include any technique of using computer software to view the inside of any section of the gastrointestinal tract, including CT imaging, MR imaging, PET imaging, or the like. Such medical or diagnostic procedures may also include fiberoptic endoscopy, optical colonoscopy, sigmoidoscopy and the like, and combinations thereof.

In one embodiment, the invention may include a system of distending the colon in conjunction with MR imaging of the colon or other organs requiring such distention. The system may comprise an electro-pneumatic insufflator, accessory cart and distention media supply cylinders in a location, such as a shielded MRI control room, that is shielded from the magnetic field of the MRI equipment. In one embodiment, the insufflator may be disposed in a room that is adjacent to a MRI equipment room in which the MRI scanner is located. As a result, the electro-pneumatic insufflator and any accompanying equipment can be disposed in a location, where they are not affected by the magnetic force of the MRI scanner. In the invention, the system comprises an electromagnetically inactive connection tube fabricated from non-ferromagnetic materials. In one embodiment, the connection tube may have a proximal end connected to a consumable outlet connection of the insufflator and a distal end terminating in the MRI equipment room. In one embodiment, the electromagnetically inactive connection tube may pass through the shielded enclosure of the MRI equipment room along its length between its proximal and distal ends. In one alternative embodiment, the distal end of the electromagnetically inactive connection tube may terminate with a connector receptacle fabricated from non-ferromagnetic materials that is connectable to a consumable administration set. In another alternate embodiment, the electromagnetically inactive connection tube itself is connectable to the patient.

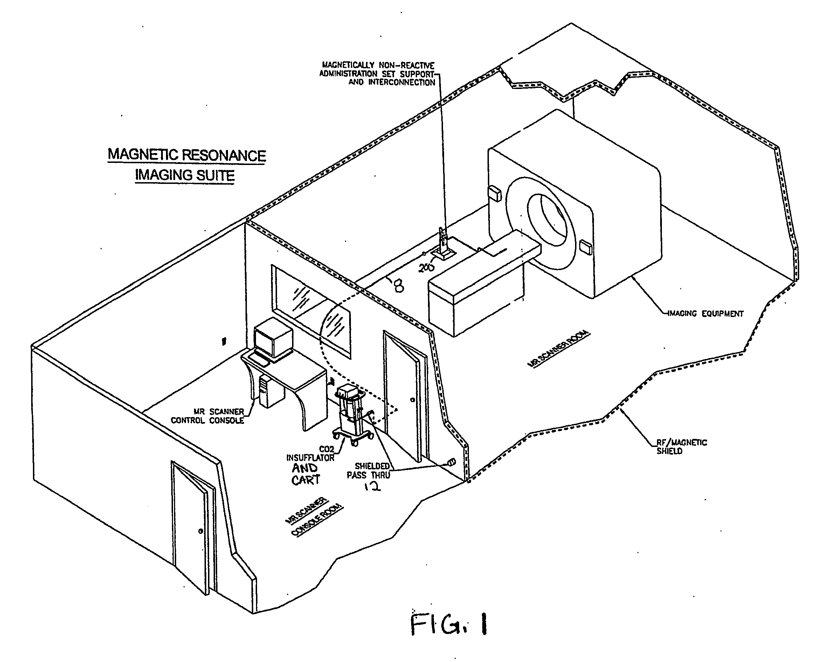

Referring to **FIG.1****,** an illustration of an embodiment of the MRI suite showing the boundaries of the rooms of the invention, is provided. In this embodiment, the MRI scanner console room, also known as the MRI control room, contains the MRI scanner control console, the electro-pneumatic carbon dioxide insufflator, the carbon dioxide supply cylinder, and the accessory cart. A technician can monitor the scanning operation and manipulate the scanning process by way of the MRI scanner control console. The electromagnetically inactive connection tube **8** passes through either of the shielded pass-throughs, waveguide or utility openings **12** so that it can enter the MRI scanner room, also referred to herein as the MRI equipment room. The MRI scanner is shown at the end of the MRI equipment room that is farthest away from the insufflator and MRI scanner control console. The patient rests on the table in front of the imaging equipment, and a support stand **200** is shown on one side of the table. The dotted line enclosing the MRI equipment room denotes a shield that keeps electromagnetic and RF radiation from traversing the MRI equipment room, which limits any outside interference with the equipment in the MRI control room and thereby improves the safety and accuracy of the MRI for the reasons explained above.

In one embodiment, a support stand comprising non-ferromagnetic and electrically nonconductive materials secures and positions the electromagnetically inactive connection tube from the insufflator in a favorable position for attaching the connector receptacle to the consumable administration set. For example, the support stand can hold a portion of the tubing off of the floor to prevent kinks from forming in the electromagnetically inactive connection tube. The support stand optionally has features that secure and position an effluent trap belonging to consumable administration set in a favorable position to promote the drainage of the patient's effluent into the effluent trap. In one embodiment, the support stand may position the electromagnetically inactive connection tube and the components of the consumable administration set lower than the patient so that effluent may drain to the effluent trap via gravity. In some embodiments, the support stand may include an attachment device for the effluent trap so that the effluent trap is maintained in an upright position. The height for supporting the tubing on the support stand may be from about 12-36 inches (304.8 - 914.4mm) above floor level. As such, the height of the support stand may be from 12-36 inches (304.8 - 914.4 mm).

In one embodiment, the support stand may be universally movable within the shielded MRI equipment room. In some embodiments, the support stand may be constructed to be small and lightweight but capable of maintaining an upright position and supporting the weight of the electromagnetically inactive connection tube and the consumable administration set. In addition, the support stand may include one or more features, such as a handle or non-magnetic casters, to facilitate ease of location or relocation in MRI equipment room or in other areas. The support stand may be constructed of multiple pieces that may be easily assembled and disassembled.

Also disclosed is a method of insufflation of the colon wherein all ferromagnetic and electrically active components are located outside of the shielded MR imaging equipment room. In this method, the user can operate all electrically active components outside of the shielded MRI equipment room and make a non-ferromagnetic and electrically inactive pneumatic connection from the active components into the shielded MRI equipment room to the patient undergoing an MRI exam.

In one alternative embodiment, the insufflator of the invention may be an automatic insufflator unit. Automatic insufflator units suitable for use herein may include, but are not limited to, any electronic device for displacing gas into the organ to be imaged (such as the colon). The distension medium used to distend the organ to be imaged may be a gas suitable for administration in the body, such as carbon dioxide or air. In one embodiment of the invention, the unit is an electro-pneumatic carbon dioxide insufflator adapted to distend the colon, such that the unit delivers CO₂ to the patient's colon for distention by specifying the following parameters at the control interface. When rectally inserting an enema tip of the consumable administration set in a patient, as explained below, an appropriate distention pressure of CO₂ may include, for example, 0 to 25 mm Hg. Set flow rates of CO₂ may include about 1-20 L/mm, and set pressure from about 10 mm Hg to about 50 mm Hg, for example about 3 to 6 L/mm, and 20-30 mm Hg, respectively.

In one embodiment, the insufflator of the present invention is an E-Z-EM PROTOCO₂L™ Colon Insufflator used to displace and regulate CO₂ as a distention media to a patient's colon for purposes of colonography, or any other diagnostic procedure requiring colon distention. The PROTOCO₂L™ Colon Insufflator is commercially available and is based on currently marketed laparoscopic insufflator technology. This insufflator unit is a software controlled electromechanical system that precisely regulates pressure and meters flow of CO₂ from a supply cylinder to the patient.

In one alternative embodiment, the electromagnetically inactive connection tube of the present invention may be in the form of one or more hollow areas capable of conveying the distension medium from one location to another. For example, in one embodiment of the present invention, the electromagnetically inactive connection tube may include a structure that comprises one or more hollow areas, and is capable of conveying the distension medium or otherwise functions as a passageway for the distension medium. The electromagnetically inactive connection tube may include, but is not limited to, a hollow cylinder such as a flexible tube, channel, or pipe. The electromagnetically inactive connection tube may also comprise a single lumen or multilumens.

In one embodiment, the electromagnetically inactive connection tube may be constructed of any suitable electrically inactive and non-ferromagnetic material. In some embodiments, the electromagnetically inactive connection tube may be constructed of an elastomeric material, such as olefin-based materials, including but not limited to, polyethylene, ethylene, ethylene-propylene copolymers, ethylene-vinyl acetate copolymers, ethylene-acrylic ester copolymers, and combinations thereof. The electromagnetically inactive connection tube may also be constructed of polyvinyl chloride or ethylene-vinyl alcohol copolymers, polyvinyl chloride, polyester, polyamide or polyurethanes, silicone, rubber, nylon, or PTFE. Exemplary materials for construction of the electromagnetically inactive connection tube include PTFE, polyurethane, and silicone.

In one embodiment, at least one portion of the electromagnetically inactive connection tube may comprise a connection means. Such connection means may include, but is not limited to, means for performing a connection with one or more components. In one alternative embodiment, the connection means includes a Luer connection, Colder connection, barbed connection, male/female connection or any equivalent thereof. In one alternative embodiment, the connection means provides means for forming a fluid-type seal between one or more portions of the electromagnetically inactive connection tube and an insertable member, opening of the effluent trap or one or more apparatuses used in the medical or diagnostic procedure. The tubular portion of the electromagnetically inactive connection tube and any distal connection means must be constructed of electromagnetically inactive and non-ferromagnetic material. In one embodiment, the proximal connection means may also be constructed of electromagnetically inactive and non-ferromagnetic materials. However, as the proximal connection means is connectable to the insufflator which is shielded from the MR scan, such proximal connection means may include electrically active and/or ferromagnetic materials.

The electromagnetically inactive connection tube may be long enough such that it can extend two rooms yet not so long that the flow of the distension medium is impeded. Specifically, the electromagnetically inactive connection tube can be long enough to connect from the insufflator when located in a first room to either a patient undergoing MR scanning located in a second room or to one or more components of a consumable administration set located near a patient undergoing MR scanning in a second room. Additionally, the diameter of the tube may be a standard diameter used with an insufflator, such as ¼ inch (6.35 mm) to ½ inch (12.7 mm) inner diameter, or for example about 5/16 inch (7.9375 mm) standard outer diameter tubing. In one embodiment, an electromagnetically inactive connection tube having a diameter from about ¼ inch (6.35 mm) to about ½ inch (12.7 mm) diameter may be capable of extending to great lengths, including up to 55 feet (16764 mm), without degradation of distension medium flow performance due to frictional losses. The length of the electromagnetically inactive connection tube may be from about 25 feet (7620 mm) to about 55 feet (16764 mm), or from about 35 feet (10668 mm) to 50 feet (15240 mm), for example.

Due to cost prohibitions, electro-pneumatic insufflators are not themselves consumable and may be used with multiple individuals. In this regard, it may be desirable that the insufflator and equipment used therewith not be contaminated from use by any other patient. Effluent, such as stool, may be expelled from the patient during the distension procedure, and can contact thus contaminating the insufflator and associated equipment. Therefore, a consumable administration set with, for example, a hydrophobic filter or barrier can be used with the insufflator to prevent contact with the patient and the patient's effluent and to prevent cross-contamination among patients. In one embodiment, the components of the consumable administration set in direct contact with the patient may be provided in latex free form to prevent allergic reaction of the patient. In some embodiments, all components of the consumable administration set are provided in latex free form to prevent incidental contact and allergic reaction of the patient.

In several embodiments, the consumable administration set comprises a consumable connection tube, which, like the electromagnetically inactive connection tube, is also electrically inactive and non-ferromagnetic. In these embodiments, the distal end of the electromagnetically inactive connection tube may terminate with a connector receptacle fabricated from non-ferromagnetic materials that may be connectable to at least one component of the consumable administration set. For example, in one embodiment, the consumable administration set further comprises an effluent trap, also referred to herein as an effluent reservoir. In this embodiment, the electromagnetically inactive connection tube may be connectable to the effluent trap, and the effluent trap may be connectable to the consumable connection tube. In another embodiment, the electromagnetically inactive connection tube is connectable to the consumable connection tube.

In an alternate embodiment, the electromagnetically inactive connection tube itself is consumable and is connectable to both the patient and the insufflator. In this embodiment, an additional consumable administration set or additional components, such as barriers or effluent traps, may optionally be used. In another alternate embodiment, the electromagnetically inactive connection tube is connectable to a first consumable connection tube. The first consumable connection tube in this embodiment may be connectable to another component of the consumable administration set, such as an effluent trap or a second consumable connection tube.

In one embodiment, the consumable administration set may further comprise an insertable member. The insertable member may be integral with the consumable connection tube or may be a separate component that is connectable, directly or indirectly, to the consumable connection tube. The insertable member, when present in an embodiment, is suitable for insertion into an opening of a cavity of an individual so that the distension medium may enter an organ or organs of the individual. The insertable member may have one or more hollow areas, such as a multilumen tube, for example. The insertable member may include, but is not limited to, an instrument for examining the interior of the individual's cavity, such as a trocar, endoscope, enema tip, Foley catheter, entry needle, for example. In some embodiments, the insertable member may further include an instrument for removing liquid, gas or solid from the interior of an individual's cavity.

In one alternative embodiment, the insertable member may have a front portion and a rear portion, the rear portion having one or more connection means. Such connection means includes, but is not limited to, means for forming a connection with one or more other components. In one alternative embodiment, the connection means includes, but is not limited to, means for forming a Luer connection, Colder connection, barbed connection, male/female type connection or any equivalent thereof. In one alternative embodiment, the connection means provides means for forming a fluid-type seal between a hollow area of an insertable member with one or more conduits or openings of the effluent reservoir.

The insertable member may be inserted in the organ or body part to be distended and scanned by the MR scanner, and therefore may be constructed from electromagnetically inactive and non-ferromagnetic material when used in this manner. In one embodiment, the insertable member may comprise a solid, substantially rigid material. Such materials may also include PVC or polyethylene, for example. It may also comprise a substantially resilient material, such as rubber or an elastomeric polymer, such as a soft plastic, polyurethane, latex, nylon, vinyl, PTFE, silicone or a blend thereof.

The effluent trap, or effluent reservoir, may be included in the consumable administration set. The effluent reservoir may comprise a hollow interior capable of receiving and collecting effluent that passes through an opening of an individual's internal cavity during or after a diagnostic or medical procedure, for example. The effluent reservoir is particularly useful as a reservoir for collecting effluent from an individual's body cavity, thus preventing it from reentering the body cavity or contaminating a component, device or apparatus used in connection with a medical or diagnostic procedure.

In one embodiment, the effluent reservoir may comprise an interior area having a closed bottom, and front and rear walls secured together around their periphery. The reservoir may also comprise one or more ports or openings for admitting or removing effluent to the interior of the effluent reservoir. The reservoir may further include one or more ports or opening for use in conveying a desired medium through the interior of the effluent reservoir. The reservoir may hold about 10 cc to 1000 cc of fluid, or 10 cc to 500 cc of fluid, preferably about 10 cc to 100 cc, more preferably about 60 cc to 100 cc. In one alternative embodiment, the effluent reservoir may hold approximately 60 cc or 100 cc of fluid, respectively.

In one embodiment, the effluent reservoir has a bag-like shape. Alternatively, it may have a bottle-like, tray-like, boxlike, or tube-like shape, for example. In another embodiment, the effluent reservoir may comprise a rigid container or jar, or it make take the form of a collapsible container. One advantage of a collapsible container is its smaller material volume which facilitates handling during manufacture, storage, shipping, use and disposal.

The effluent reservoir in one embodiment as used in proximity to the MR scanner should be constructed from non-ferromagnetic and non-electrically conductive material. The effluent reservoir may be prepared from suitable plastic material whereby a strong, lightweight, reliable, yet economic container is provided. For example, the effluent reservoir of the present invention may be constructed of any suitable elastomeric material, such as olefin-based materials, including but not limited to, polyethylene, ethylene-propylene copolymers, ethylene-vinyl acetate copolymers, ethylene-acrylic ester copolymers, iononomers, and combinations thereof. Furthermore, film layers of polymers having barrier properties, such as polyvinylidene chloride and ethylene-vinyl alcohol copolymers, as well as film layers of such polymers as polyvinyl chloride, polyester, polyamide and polyurethanes may also be used.

The effluent reservoir may also comprise any flexible material, including polyethylene film, plasticized polyvinyl chloride film, plasticized polyvinylidene chloride film polyethylene/ethylene-vinyl acetate copolymer laminate, ethylene-vinyl acetate copolymer/polyvinylidene chloride/ethylene-vinyl acetate copolymer laminate, and polyethylene/ethylene-vinyl acetate copolymer/polyethylene chloride/ethylene-vinyl acetate copolymer/polyethylene laminate, among others. Also, the effluent reservoir may comprise materials that make it suitable for disposal in a flush toilet. Such materials comprising a biodegradable polymer, for example.

The consumable administration set if included in an embodiment may optionally include other components, including but not limited to one or more restraining means to maintain the insertable member in a desired position once inserted through the opening of the individual's body cavity or prevent the tip of the insertable member from being displaced after insertion into the individual's body cavity. The PROTOCO₂L™ Administration Set, commercially available from E-Z-EM, Inc., is a consumable administration set that may be used with a PROTOCO₂L™ Colon Insufflator. The PROTOCO₂L™ Administration Set includes an eight foot consumable connection tube. In addition to the consumable connection tube, the PROTOCO₂L™ Administration Set comprises two balloon inflators, a plastic tubing clamp, Flexi-Tip with Flexi-Cuff silicone elastomer retention cuff, 0.1 micrometer hydrophobic filter, 100 mL effluent collection container, and a connector to PROTOCO₂L™ Colon Insufflator. Alternatively, the PROTOCO₂L™ Administration Set may be used with any pneumatic manual insufflator, including the E-Z-EM hard bulb or E-Z-EM E-Z-Flat device, sold by E-Z-EM, Inc., Westbury, New York.

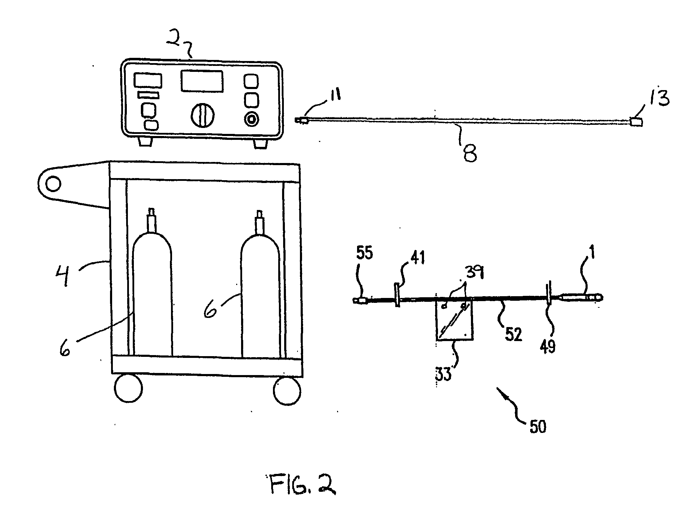

Referring to **FIG. 2****,** an illustration of one alternative embodiment of the system for distending an organ is provided. An electro-pneumatic insufflator 2 rests on top of an accessory cart 4, which is stored in an MRI control room. Various controls are depicted on the insufflator in the form of boxes on the front of the device. One or more distention medium, e.g., CO₂, supply cylinders **6** may be stored in the accessory cart **4.** In one embodiment, the electromagnetically inactive connection tube **8** may be connectable to the insufflator **2** at its proximal end with a connector **11.** The consumable administration set **50** generally comprises a consumable connection tube **49,** which may also be electrically inactive and non-ferromagnetic. The consumable administration set **50** may optionally include other disposable components such as one or more barriers **41, 49,** an effluent reservoir **33,** an insertable member **1,** and one or more connectors **55,** the totality of which is electromagnetically inactive itself. As demonstrated by the arrow shown in **FIG. 2****,** the connection tube may extend beyond the area shown so that it can be used to inflate the patient's gastrointestinal tract.

Possible components of the consumable administration set are described in U.S. Patent Application No. 10/497,625, which was published as Patent Publication No. 2005-0038374 A1, and is incorporated by reference herein in its entirety. The effluent reservoir **33** of the consumable administration set may comprise a hollow area having a closed bottom and front and rear walls secured together around their periphery. The reservoir **33** may also comprise one or more ports **39** for admitting or removing effluent to the interior of the effluent reservoir. The reservoir **33** may further include one or more ports **39** for use in conveying a medium to or from the interior of the effluent reservoir. The effluent reservoir **33** may be a rigid or collapsible container, preferably a collapsible container.

The consumable administration set may also comprise one or more barriers **41, 49.** The one or more barriers **41, 49** may be positioned at one or more sites including, but not limited to, any location between the effluent reservoir **33** and the area to be protected from contacting the individual's effluent. For example, one or more barriers **41** may be positioned in order to prevent effluent from contacting, and thus contaminating a component, device or apparatus used in connection with a medical or diagnostic procedure, such as the insufflator. Also, the one or more barriers may be positioned in various locations in order to prevent the effluent or medium migrating from the effluent reservoir **33** through the insertable member **1** and into the individual's internal cavity. In one alternative embodiment, the barrier **41** may comprise one or more layers of material impervious to the passage of water, but not gas. Such a barrier may materially reduce the transfer of pathogens, such as viruses and bacteria, mucous and fluid. In one alternative embodiment, the effluent barrier **41** may comprise a hydrophobic membrane to provide an anti-viral and anti-bacterial barrier, including but not limited to a 0.1 micron hydrophobic membrane. In another embodiment, the adjustable barrier may include a clamp, valve, stop-cock, locking pinch clamp **49.**

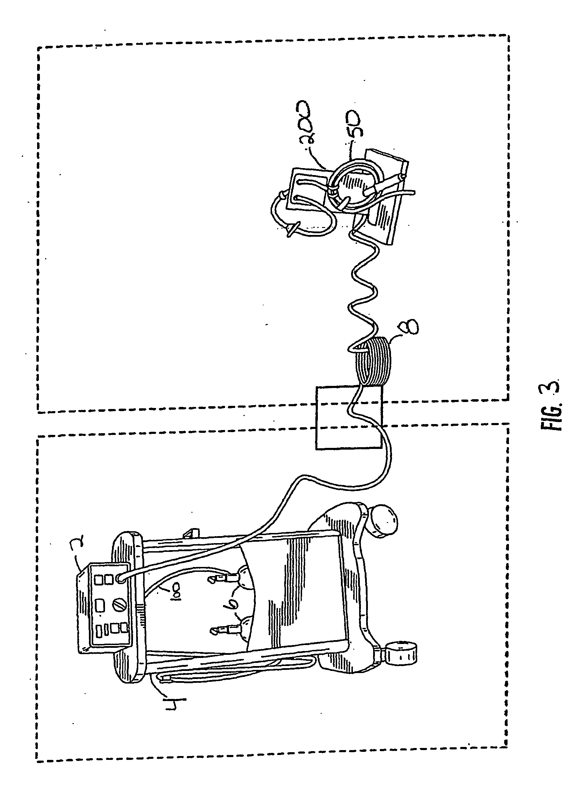

**FIG. 3** shows a further view of an embodiment of a system of the invention. **FIG. 3** more clearly shows the connection of the carbon dioxide supply cylinders **6** to the electro-pneumatic insufflator **2** through the use of one or more carbon dioxide connection tubes **100.** Unlike the electromagnetically inactive connection tube **8** that connects the insufflator to the patient, the one or more carbon dioxide connection tubes **100** need not be electromagnetically inactive. The dotted lines forming two boxes around the equipment demonstrate that the electromagnetically inactive parts of the system have to be separated from the electromagnetically active parts. In an embodiment of the MRI suite of the invention, the parts on the left are electromagnetically active and are located in the MRI control room. The electromagnetically inactive connection tube **8** is fed through the wall or other structure separating the parts of the system, and it is held in place in the MRI equipment room by the support stand. The electromagnetically inactive connection tube **8** can extend for great lengths, and a tube length of 45 feet (13716 mm) to 55 feet (16764 mm) has been shown to work very well for purposes of distending the colon. The electromagnetically inactive connection tube is held off of the floor in the shielded area by a support stand **200.** The support stand **200** may also hold components of the consumable administration set **50.**

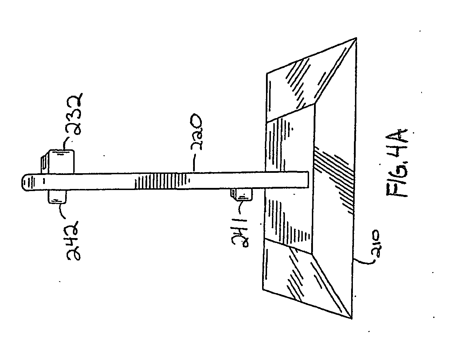

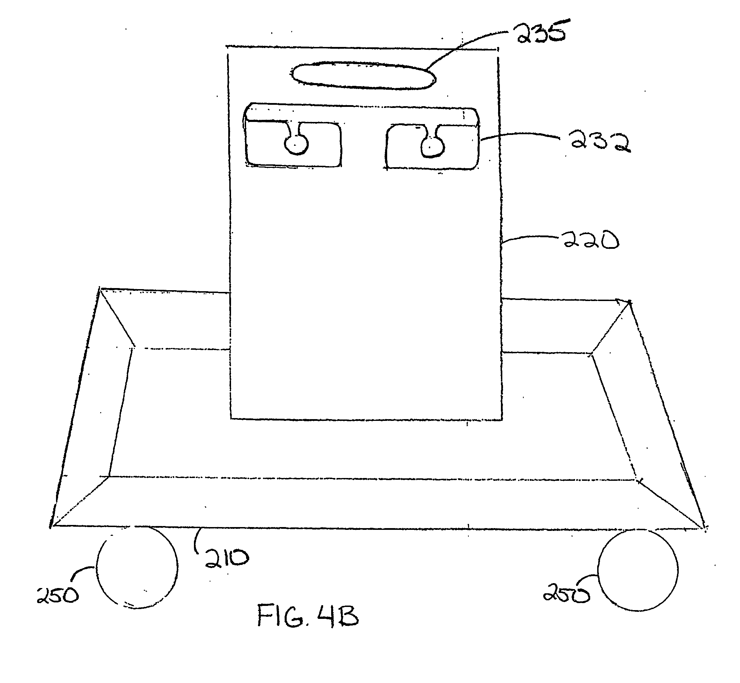

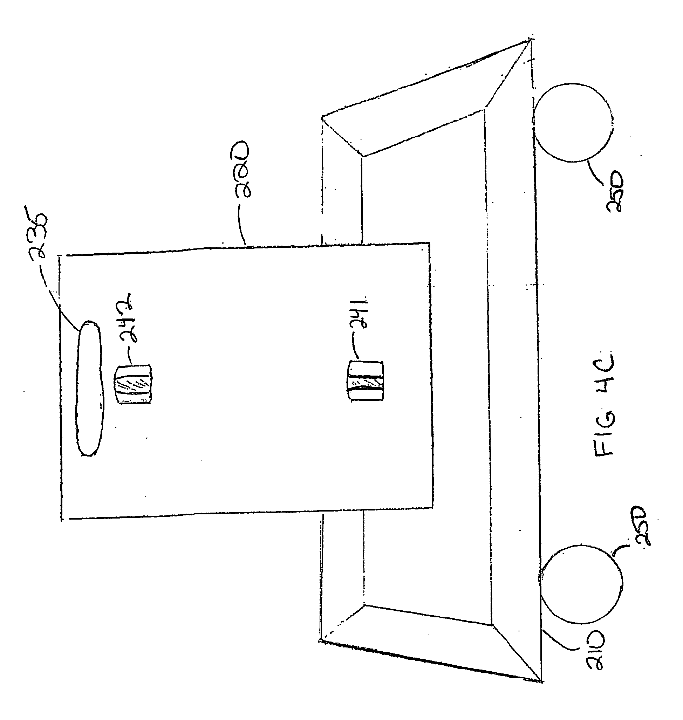

Several alternative embodiments of the invention may include a support stand. One embodiment of the support stand is shown in **FIGS. 4A****,** **4B**, and **4C****.** The support stand of this embodiment is used in the shielded area, such as an MRI equipment room, containing the MRI magnet, and thus the support stand must be made of electrically inactive/non-ferromagnetic material. The support stand as depicted in **FIGS. 4A****,** **4B**, and **4C** has a rectangular base **210** that ensures that the support stand is not easily upset or toppled over. However, the base of the support stand may be in any shape that supports the support appendage and is not easily upset or topped over, including but not limited to circular, oval, or square bases. The support appendage **220** extending from the base of the support stand has several notches **241, 242** in which the electromagnetically inactive connection tube can be placed. Placing the connection tube in the notches allows the tube to be manipulated easily by a technician, and it also keeps the tube from moving in an unpredictable and potentially dangerous manner around the MRI equipment. The support stand of this embodiment as shown in **FIG. 4B** also includes a clip or set of clips **232** for holding the effluent trap. The support stand also may include a handle **235** and/or electrically inactive and non-ferromagnetic casters **250.** The handle **235** may be in the form of a void in the support appendage large enough to fit a hand through, as shown in the Figures, or may be an additional appendage on the top or side of the support appendage **220.**

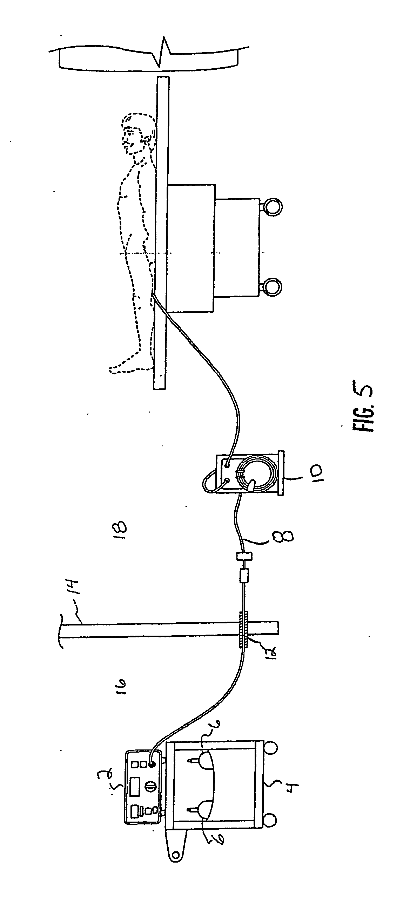

**FIG. 5** is a further illustration of one embodiment of an MRI suite and distension system according to the invention. An electro-pneumatic insufflator **2** rests on top of an accessory cart **4.** As shown in **FIG. 5****,** supply cylinders **6** for distension medium such as carbon dioxide rest in the accessory cart **4.** The electro-pneumatic insufflator **2** suitable for use with the present invention may include, but is not limited to, the E-Z-EM PROTOCO₂L™ Colon Insufflator or a similar device, which is sold by E-Z-EM, Inc., Westbury, New York.

The supply cylinders **6** supply carbon dioxide or another distension medium to insufflator **2,** and the insufflator **2** forces the carbon dioxide through an electromagnetically inactive connection tube **8** that connects to the patient's body through the patient's rectum. The electromagnetically inactive connection tube **8** is held in place by a support stand **10** that elevates the electromagnetically inactive connection tube **8** and prevents it from forming kinks or being damaged in other ways so that the insufflator **2** can function properly.

**FIG. 5** also shows the necessary separation by a shield **14** of the electromagnetically active components in the MRI control room **16** and the non-electromagnetically active components in the MRI equipment room **18** along with the patient and the MRI equipment (not shown). If the electromagnetically active components were in the same room as the powerful magnetic forces of the MRI, the gastrointestinal imaging process could be disturbed, and the metal components of the insufflator **2** or canisters **6** could become projectiles and cause great harm to the patient or others present at the time. Furthermore, the RF waves from the MR scanner could disturb various components of the insufflator and prevent the insufflator from providing sufficient pressure of distension medium to properly distend the colon or other organs to be imaged. The shield **14** or Faraday cage can be made of any material that blocks the magnetic, namely RF, radiation so that it does not cause the electromagnetically active components external to the shield to be affected by the radiation. In one embodiment, the shield **14** may comprise a partition or wall made of non-ferromagnetic but electrically conductive materials. The shield may be made of copper plate and/or copper mesh. The shield has at least one utility opening **12** through which the electromagnetically inactive connection tube can be routed. The opening **12** is large enough to allow the connection tube to pass through, but it is sufficiently small so that the force of the magnet and associated RF energy of the MRI scanner cannot affect the accessory cart **4** or any other electric or ferromagnetic material on the other side of the shield **14.** Additionally, the material forming the at least one utility opening **12,** may be in conductive contact with the rest of the shield and should be tuned to the frequencies of the MR scanner. The material forming the at least one utility opening may be constructed with a diameter and length such that it acts as a waveguide and does not resonate at the operating frequencies of the MR scanner to maintain isolation of the radio waves.

Also annotated on **FIG. 5** are the boundaries where these respective components could be situated in the MRI suite. To deploy the system of the invention, the extension tube would need to be routed through one of the utility openings from the MR control room to the shielded MR imaging equipment room.

### EXAMPLES

The following example details a feasibility assessment that was performed pertaining to the potential use of a PROTOCO₂L™ Colon Insufflator System in an MRI environment for an MR colonography study. The assessment was limited to comparative measurements of the flow and pressure performance of the PROTOCO₂L™ Insufflator and associated devices under experimental conditions first representing CT colonography and secondly representing MR colonography.

The first experimental set-up representing CT colonography included a measured in-vitro fill into a 3 Liter collapsible container through the standard administration set connected to the PROTOCO₂L™ Insufflator. The second experimental set-up representing MR Colonography included a measured in-vitro fill into the same 3 Liter collapsible container through PROTOCO₂L™ Administration Set which in turn was connected to a 15.2 meter (50 ft.) electromagnetically inactive connection tube of like diameter to that of the PROTOCO₂L™ Administration Set. The use of the electromagnetically inactive connection tube enables remote delivery of carbon dioxide from a PROTOCO₂L™ Insufflator situated in the MRI control room to the patient in the MRI equipment room. For both experimental set-ups, a TSI recording anemometer type (i.e., mass flow meter) flow meter/pressure gauge was connected at the end of the administration set lumen where an enema tip fitting is normally connected. Attached to the outlet of the TSI instrument was the 3 Liter collapsible effluent reservoir (Super XL Enema Bag). This experimental arrangement placed the recording device near the patient's rectum. Thus, the effects of pneumatic compliance associated with the 15.2 meter electromagnetically inactive connection tube for the MRI variant were measured for this initial assessment. Additionally, for both experimental set-ups, the effluent trap was left in its normal configuration. For the MRI experimental set-up, the 15.2 meter electromagnetically inactive connection tube simply consisted of a continuous length 8 mm (5/16") diameter PVC tubing terminated with a male and female Colder fitting to connect the administration set to the PROTOCO₂L™ insufflator device.

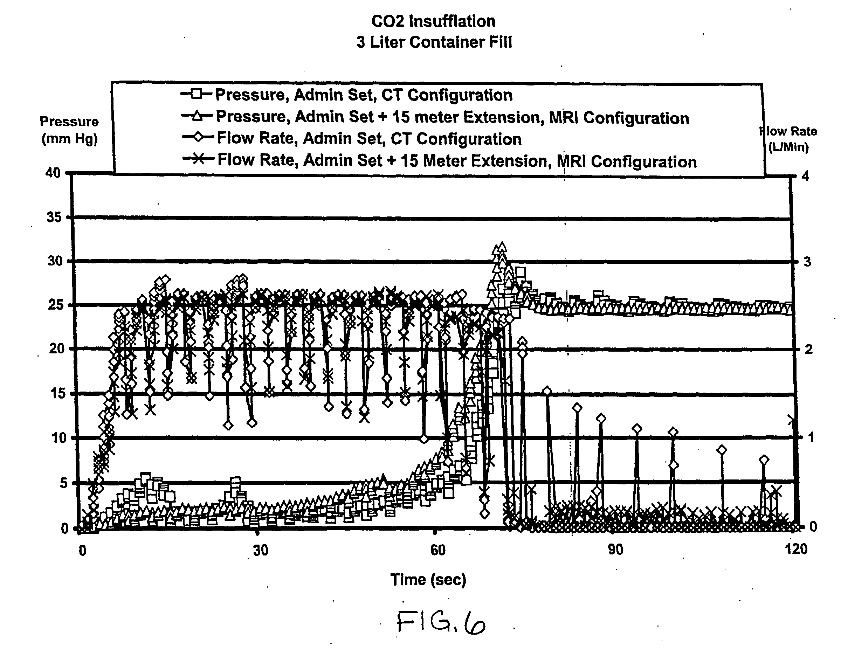

Pressure and flow rate time histories were acquired via the TSI flow/pressure meter connected with the PROTOCO₂L™ Administration Set outlet. Results for both experimental conditions were plotted on the same graph shown in **FIG. 6****.** For both experimental conditions, data was recorded for 120 seconds, allowing the system to fully fill the collapsible reservoir. At the conclusion of data acquisition, the collapsible containers were manually squeezed to verify electronic pressure relief performance.

Per the graph shown in **FIG. 6****,** flow and pressure performance for the 3 Liter fill are for all practical purposes identical with and without the 15.2 meter extension. This would indicate that displacing carbon dioxide to a patient's colon remotely through an extension tube is feasible and should not be significantly different than that of the current CT performance.

Upon squeezing the collapsible container, the electronic pressure release valve opened when the pressure exceeded the set pressure by 2 mm Hg. For the case where the 15.2 meter extension was used, its lumen volume of 0.75 Liter did not have any noticeable impact on overall compliance when squeezing the 3 Liter test container to the point of venting. The extension does not significantly impact flow/pressure performance.

Proposed use of an extension for MRI colonography purposes should additionally consider MRI compatibility. Particularly, the female end of the extension tube used in the MRI equipment room adjacent to the magnet should be void of any ferromagnetic materials.

Other modifications and other embodiments of the invention set forth herein will come to mind to one skilled in the art to which this invention pertains having the benefit of the teachings presented in the foregoing descriptions and the associated drawings. Therefore, it is to be understood that the invention is not to be limited to the specific embodiments disclosed. Although specific terms are employed herein, they are used in a generic and descriptive sense only and not for purposes of limitation.

Further, throughout the description, where compositions are described as having, including, or comprising specific components, or where processes or methods are described as having, including, or comprising specific steps, it is contemplated that compositions of the present invention also consist essentially of, or consist of the recited components, and that the processes or methods of the present invention also consist essentially of or consist of the recited steps. Further, it should be understood that the order of steps or order for performing certain actions are immaterial so long as the invention remains operable. Moreover, two or more steps or actions may be conducted simultaneously with respect to the invention disclosed herein.

## Claims

1. A magnetic resonance imaging (MRI) suite for performing MRI scanning of one or more organs requiring distention, the MRI suite comprising:
a separate shielded area having a shield (14) that substantially blocks magnetic and radio frequency (RF) radiation,
an area outside the shielded area,
an MRI scanner, and
a system for distending the one or more organs, the system comprising:
an insufflator (2), and
an electromagnetically inactive connection tube (8) fabricated from non-ferromagnetic materials ; and
wherein the insufflator is located in the area outside the shielded area, the MRI scanner is located in the separate shielded area, and wherein one end of the electromagnetically inactive connection tube is located in the area outside the shielded area and the other end of the electromagnetically inactive connection tube is located in the separate shielded area.

2. The MRI suite of claim 1, wherein the separate shielded area comprises an MRI equipment room and wherein the area outside the shielded area comprises an MRI control room.

3. The MRI suite of claim 1, the system further comprising
a support stand (200) fabricated from non-magnetic materials that provides support for the electromagnetically inactive connection tube.

4. The MRI suite of claim 1, wherein the insufflator further comprises a consumable outlet connection connectable to a consumable administration set.

5. The MRI suite of claim 4, wherein the electromagnetically inactive connection tube has its proximal end connected to the consumable outlet connection of the insufflator, the electromagnetically inactive connection tube passing through the shield along a span between the electromagnetically inactive connection tube's proximal and distal ends, the distal end of the electromagnetically inactive connection tube located with the separately shielded area.

6. The MRI suite of claim 1, wherein the electromagnetically inactive connection tube is between 45 feet (13716 mm) and 55 feet (16764 mm) long.

7. The MRI suite of claim 3, wherein the distal end of the electromagnetically inactive connection tube is terminated with a connector receptacle.

8. The MRI suite of claim 7, wherein the connector receptacle comprises non-magnetic materials.

9. The MRI suite of claim 8 wherein the connector receptacle is connected to an electromagnetically non-reactive consumable administration set (50), the consumable administration set comprising a consumable connection tube (89), an enema tip positioned at one end of the consumable connection tube wherein the tip is insertable into the rectum of a patient; an inflatable balloon located on the enema tip for preventing gas from escaping through the rectum; a flexible trap connected to said tubing for collecting effluent or stool expelled from the patient; and a hydrophobic filter positioned in-line to said tubing positioned proximally from the enema tip.

10. The MRI suite of claim 3, wherein the support stand secures the electromagnetically inactive connection tube from the insufflator in a favorable position for attaching the connection receptacle to a consumable administration set.

11. The MRI suite of claim 10, further comprising an effluent trap connectable to the electromagnetically inactive connection tube and a consumable connection tube connectable to the effluent trap, wherein the support stand and the effluent trap are set in position to promote the drainage of patient effluent into the effluent trap.

12. The MRI suite of claim 3, wherein the support stand is universally movable within the separate shielded area.

13. The MRI suite of claim 12, wherein the support stand possesses features to facilitate ease of location of the MRI equipment.

14. The MRI suite of claim 12, wherein the support stand further comprises a handle (235).

15. The MRI suite of claim 12, wherein the support stand further comprises non-magnetic casters (250).

16. A system for distending one or more organs in a patient located in a first
room, the system comprising:
an insufflator (2),
an insufflation medium, and **characterised by**
an electromagnetically inactive connection tube (8) fabricated from non ferromagnetic materials for transmitting the insufflation medium to the patient's one or more organs,
wherein the electromagnetically inactive connection tube is long enough to extend from the insufflator when located in a second room to the patient located in the first room while maintaining adequate flow of the insufflation medium,
wherein the electromagnetically inactive connection tube is 15 to 55 feet (4572 mm to 16764 mm) long.

17. The system as claimed in claim 16, wherein the electromagnetically inactive connection tube is adapted to pass through a RF shield (14).

18. The system as claimed in claim 16, further comprising a support stand (210) for supporting the electromagnetically inactive connection tube.

19. The system as claimed in claim 16, further comprising a consumable administration set connectable to the electromagnetically inactive connection tube.

## Patentansprüche

1. Magnetresonanztomographie (MRI) Suite zur Durchführung des MRI-Scannens von einem oder mehreren Organen, die eine Ausdehnung erfordern, wobei die MRI-Suite umfasst:
einen separaten abgeschirmten Bereich, der eine Abschirmung (14) aufweist, die magnetische und Hochfrequenz-(RF) Strahlung im Wesentlichen abblockt,
einen Bereich außerhalb des abgeschirmten Bereichs,
einen MRI-Scanner und
ein System zur Ausdehnung des oder der Organe, wobei das System umfasst:
einen Insufflator (2) und
ein elektromagnetisch inaktives Verbindungsrohr (8), hergestellt aus nicht-ferromagnetischen Materialien, und
wobei der Insufflator in dem Bereich außerhalb des abgeschirmten Bereichs angeordnet ist, der MRI-Scanner im separaten, abgeschirmten Bereich angeordnet ist, und wobei ein Ende des elektromagnetisch inaktiven Verbindungsrohrs im Bereich außerhalb des abgeschirmten Bereichs angeordnet ist und das andere Ende des elektromagnetisch inaktiven Verbindungsrohrs in dem separaten abgeschirmten Bereich angeordnet ist.

2. MRI-Suite nach Anspruch 1, in der der separate abgeschirmte Bereich einen MRI-Geräteraum aufweist und wobei der Bereich außerhalb des abgeschirmten Bereiches einen MRI-Kontrollraum aufweist.

3. MRI-Suite nach Anspruch 1, wobei das System weiter einen Aufnahmeständer (200) umfasst, der aus nicht magnetischen Materialien hergestellt ist, welcher eine Abstützung für das elektromagnetisch inaktive Verbindungsrohr bietet.

4. MRI-Suite nach Anspruch 1, in der der Insufflator weiter einen verbrauchbaren Auslassstutzen aufweist, der mit einem verbrauchbaren Verabreichungsset verbunden werden kann.

5. MRI-Suit nach Anspruch 4, in der das proximale Ende des elektromagnetisch inaktiven Verbindungsrohrs mit dem verbrauchbaren Auslassstutzen des Insufflators verbunden ist, dass elektromagnetisch inaktive Verbindungsrohr entlang eines Bereichs zwischen proximalem und distalem Ende des elektromagnetisch inaktiven Verbindungsrohrs durch die Abschirmung hindurch tritt, das distale Ende des elektromagnetisch inaktiven Verbindungsrohrs in dem separaten abgeschirmten Bereich angeordnet ist.

6. MRI-Suit nach Anspruch 1, in der das elektromagnetisch inaktive Verbindungsrohr zwischen 45 Fuß (13716 mm) und 55 Fuß (16764 mm) lang ist.

7. MRI-Suit nach Anspruch 3, in der das distale Ende des elektromagnetisch inaktiven Verbindungsrohrs in einer Anschlussaufnahme endet.

8. MRI-Suit nach Anspruch 7, in der die Anschlussaufnahme nicht magnetische Materialien enthält.

9. MRI-Suit nach Anspruch 8, in der die Anschlussaufnahme mit einem elektromagnetisch nicht reaktiven, verbrauchbaren Verabreichungsset (50) verbunden ist, das verbrauchbare Verabreichungsset ein verbrauchbares Verbindungsrohr (49), eine Klistierspitze, die an einem Ende des verbrauchbaren Verbindungsrohrs angeordnet ist, wobei die Spitze in das Rektum eines Patienten einführbar ist, einen aufblasbaren Ballon, der an der Klistierspitze angeordnet ist, um das Entweichen von Gas aus dem Rektum zu verhindern; einen flexiblen Abscheider, der mit dem Rohr verbunden ist, um vom Patienten ausgeschiedenen Ausfluss oder Stuhl aufzufangen; und einen hydrophoben Filter enthält, der in-line mit dem Rohr und proximal zu der Klistierspitze angeordnet ist.

10. MRI-Suit nach Anspruch 3, in der der Aufnahmeständer das elektromagnetisch inaktive Verbindungsrohr des Insufflators in einer günstigen Position zum Anbringen der Verbindungsaufnahme an ein verbrauchbares Verabreichungsset sichert.

11. MRI-Suit nach Anspruch 10, die weiter einen Ausflussabscheider, der mit dem elektromagnetisch inaktiven Verbindungsrohr verbunden werden kann, und ein verbrauchbares Verbindungsrohr enthält, das mit dem Ausflussabescheider verbunden werden kann, wobei der Aufnahmeständer und der Ausflussabscheider in einer Position angeordnet sind, die die Drainage von Patientenausflüssen in den Ausflussabscheider begünstigt.

12. MRI-Suit nach Anspruch 3, in der der Aufnahmeständer innerhalb des separaten abgeschirmten Bereichs frei beweglich ist.

13. MRI-Suit nach Anspruch 12, in der der Aufnahmeständer Merkmale aufweist, die das Anordnen der MRI-Gerätschaft erleichtern.

14. MRI-Suit nach Anspruch 12, in der der Aufnahmeständer weiter einen Griff (235) aufweist.

15. MRI-Suit nach Anspruch 12, in der der Aufnahmeständer weiter nicht magnetische Rollen (250) aufweist.

16. System zur Ausdehnung eines oder mehrerer Organe eines Patienten, der sich in einem ersten Raum befindet, wobei das System:
einen Insufflator (2),
ein Insufflationsmedium enthält und durch
ein elektromagnetisch inaktives Verbindungsrohr (18) gekennzeichnet ist, das aus nicht ferromagnetischen Materialien hergestellt ist, zum Leiten des Insufflationsmediums zu dem oder den Organen des Patienten,
wobei das elektromagnetisch inaktive Verbindungsrohr lang genug ist, um vom Insufflator bis zum Patienten zu reichen, wenn sich der Insufflator in einem zweiten Raum und der Patient sich in dem ersten Raum befindet, wobei ein ausreichender Fluss des Insufflationsmediums aufrechterhalten wird,
wobei das elektromagnetisch inaktive Verbindungsrohr 15 bis 55 Fuß (4572 mm bis 16764 mm) lang ist.

17. System wie in Anspruch 16 beansprucht, in dem das elektromagnetisch inaktive Verbindungsrohr daran angepasst ist, durch eine RF-Abschirmung (14) hindurchzutreten.

18. System wie in Anspruch 16 beansprucht, das weiter einen Aufnahmeständer (200) zur Abstützung des elektromagnetisch inaktiven Verbindungsrohrs enthält.

19. System wie in Anspruch 16 beansprucht, das weiter ein verbrauchbares Verabreichungsset enthält, das mit dem elektromagnetisch inaktiven Verbindungsrohr verbunden werden kann.

## Revendications

1. Salle d'imagerie par résonance magnétique (IRM) pour effectuer un examen par IRM d'un ou plusieurs organes nécessitant une distension, la salle d'IRM comprenant :
une zone blindée séparée comprenant un blindage (14) qui bloque sensiblement le rayonnement magnétique et radiofréquence (RF),
une zone à l'extérieur de la zone blindée,
un appareil d'IRM, et
un système pour distendre le ou les organes, le système comprenant :
un insufflateur (2), et
un tube de raccordement électromagnétiquement inactif (8) fabriqué en matériaux non ferromagnétiques,
dans laquelle l'insufflateur est placé dans la zone à l'extérieur de la zone blindée, l'appareil d'IRM est placé dans la zone blindée séparée, et dans laquelle une extrémité du tube de raccordement électromagnétiquement inactif est placée dans la zone à l'extérieur de la zone blindée et l'autre extrémité du tube de raccordement électromagnétiquement inactif est placée dans la zone blindée séparée.

2. Salle d'IRM selon la revendication 1, dans laquelle la zone blindée séparée comprend une salle d'équipement d'IRM et dans laquelle la zone à l'extérieur de la zone blindée comprend une salle de commande d'IRM.

3. Salle d'IRM selon la revendication 1, le système comprenant en outre :
un socle de support (200) fabriqué en matériaux non magnétiques qui sert de support pour le tube de raccordement électromagnétiquement inactif.

4. Salle d'IRM selon la revendication 1, dans laquelle l'insufflateur comprend en outre un raccord de sortie consommable pouvant être raccordé à un kit d'insufflation consommable.

5. Salle d'IRM selon la revendication 4, dans laquelle le tube de raccordement électromagnétiquement inactif a son extrémité proximale raccordée au raccord de sortie consommable de l'insufflateur, le tube de raccordement électromagnétiquement inactif passant à travers le blindage le long d'un tronçon situé entre les extrémités proximale et distale du tube de raccordement électromagnétiquement inactif, l'extrémité distale du tube de raccordement électromagnétiquement inactif étant placée dans la zone blindée séparée.

6. Salle d'IRM selon la revendication 1, dans laquelle le tube de raccordement électromagnétiquement inactif a une longueur comprise entre 45 pieds (13716 mm) et 55 pieds (16764 mm).

7. Salle d'IRM selon la revendication 3, dans laquelle l'extrémité distale du tube de raccordement électromagnétiquement inactif se termine par une prise de raccordement.

8. Salle d'IRM selon la revendication 7, dans laquelle la prise de raccordement comprend des matériaux non magnétiques.

9. Salle d'IRM selon la revendication 8, dans laquelle la prise de raccordement est raccordée à un kit d'insufflation consommable électromagnétiquement non réactif (50), le kit d'insufflation consommable comprenant un tube de raccordement consommable (49) ; une canule d'insufflation positionnée à une extrémité du tube de raccordement consommable, la canule pouvant être introduite dans le rectum d'un patient ; un ballonnet gonflable placé sur la canule d'insufflation pour prévenir un échappement de gaz par le rectum ; un piège souple raccordé audit tube pour collecter un effluent ou des selles évacués par le patient ; et un filtre hydrophobe positionné en ligne avec ledit tube positionné à proximité de la canule d'insufflation.

10. Salle d'IRM selon la revendication 3, dans laquelle le socle de support maintient le tube de raccordement électromagnétiquement inactif provenant de l'insufflateur dans une position favorable pour attacher la prise de raccordement à un kit d'insufflation consommable.

11. Salle d'IRM selon la revendication 10, comprenant en outre un piège à effluent pouvant être raccordé au tube de raccordement électromagnétiquement inactif et un tube de raccordement consommable pouvant être raccordé au piège à effluent, le socle de support et le piège à effluent étant placés dans une position qui facilite le drainage d'effluent du patient dans le piège à effluent.

12. Salle d'IRM selon la revendication 3, dans laquelle le socle de support est universellement mobile dans la zone blindée séparée.

13. Salle d'IRM selon la revendication 12, dans laquelle le socle de support possède des fonctions qui améliorent la facilité de positionnement de l'équipement d'IRM.

14. Salle d'IRM selon la revendication 12, dans laquelle le socle de support comprend en outre une poignée (235).

15. Salle d'IRM selon la revendication 12, dans laquelle le socle de support comprend en outre des roulettes non magnétiques (250).

16. Système pour distendre un ou plusieurs organes d'un patient placé dans une première salle, le système comprenant :
un insufflateur (2),
un milieu d'insufflation, et **caractérisé par**
un tube de raccordement électromagnétiquement inactif (8) fabriqué en matériaux non ferromagnétiques pour transmettre le milieu d'insufflation à l'organe ou aux organes du patient,
dans lequel le tube de raccordement électromagnétiquement inactif est suffisamment long pour s'étendre depuis l'insufflateur, quand il est placé dans une seconde salle, jusqu'au patient placé dans la première salle tout en maintenant un débit adéquat du milieu d'insufflation, et dans lequel le tube de raccordement électromagnétiquement inactif a une longueur de 15 à 55 pieds (4572 à 16764 mm).

17. Système selon la revendication 16, dans lequel le tube de raccordement électromagnétiquement inactif est conçu pour passer à travers un blindage RF (14).

18. Système selon la revendication 16, comprenant en outre un socle de support (200) pour supporter le tube de raccordement électromagnétiquement inactif.

19. Système selon la revendication 16, comprenant en outre un kit d'insufflation consommable pouvant être raccordé au tube de raccordement électromagnétiquement inactif.
